# EUROPÄISCHE PATENTSCHRIFT

(11) **EP 1 269 923 B1**
(45) Veröffentlichungstag und Bekanntmachung des Hinweises auf die Patenterteilung: **11.10.2006**
(21) Anmeldenummer: 02011940.0
(22) Anmeldetag: 29.05.2002
(51) Int. Cl.: A61B 17/14, B23D 61/12

(54) **Chirurgisches Sägeblatt**
Surgical saw blade
Lame pour scie chirurgicale

(30) Priorität: 25.06.2001 DE 10131023; 25.06.2001 DE 10130007
(43) Veröffentlichungstag der Anmeldung: 02.01.2003
(73) Patentinhaber: Gebr. Brasseler GmbH & Co. KG, D-32657 Lemgo (DE)
(72) Erfinder: Küllmer, Michael, 32657 Lemgo (DE); Köster, Andreas, 32657 Lemgo (DE); Gasser, Matthias, 32791 Lage (DE); Brinkmann, Volker, 32657 Lemgo (DE)
(74) Vertreter: Weber, Joachim

(56) Entgegenhaltungen:
- EP-A- 0 551 104
- DE-A- 4 427 204
- US-A- 4 557 172
- US-A- 5 306 285
- US-A- 6 022 353

## Beschreibung

Die Erfindung bezieht sich auf ein chirurgisches Sägeblatt mit einem Einspannbereich und einem mit einer Verzahnung versehenen Arbeitsbereich.

Chirurgische Sägeblätter der beschriebenen Art sind aus dem Stand der Technik in unterschiedlichsten Ausgestaltungsformen bekannt. Sie werden an einer Antriebseinheit montiert, durch welche sie in eine oszillierende, hin und her schwingende Bewegung versetzbar sind. Hierdurch ist es möglich, beispielsweise präzise Knochenschnitte durchzuführen. Die Sägeblätter können entweder manuell geführt werden, es ist jedoch auch möglich, diese in Zusammenhang mit Schablonen zu verwenden, um auf präzise Weise vorgegebene Schnitte zu erzeugen.

Bei der Verwendung derartiger chirurgischer Sägeblätter ist es wichtig, dass die Schnitte präzise an der richtigen Stelle ausgeführt werden und dass der umgebende Knochen nicht mehr als unbedingt notwendig geschädigt wird.

Der Stand der Technik zeigt verschiedenste Konstruktionen von Sägeblättern, die mit geschränkten Zähnen versehen sind. Derartige geschränkte Sägeblätter sind jedoch nicht ausreichend präzise und weisen den Nachteil auf, dass das Sägeblatt während des Schnitts verläuft und der Schnitt auf diese Weise unpräzise wird.

Aus der US-PS 5,306,285 ist ein chirurgisches Sägeblatt bekannt, welches über nicht geschränkte Zähne verfügt. Die Zähne sind jeweils alternierend von unterschiedlichen Seiten aus angeschliffen bzw. mit einer Fase versehen und haben zudem auf jeweils einer Seite einen Hohlkehlenschliff. Die Hohlkehlenschliffe sind alternierend auf der einen bzw. der anderen Oberfläche des Körpers des Sägeblattes ausgebildet. Im Gegensatz zu den erstgenannten, geschränkten Sägeblättern, welche sich beim Einsatz sehr unruhig verhalten und ein gezieltes atraumatisches Sägen verhindern, sind Sägeblätter nach dem oben genannten US-Patent zwar etwas besser, sie verhalten sich bei der praktischen Anwendung jedoch immer noch sehr unruhig.

Die den nächstkommenden Stand der Technik bildende US-A 5,306,285 beschreibt ein chirurgisches Sägeblatt mit Sägezähnen. An dem Sägeblatt sind alternierend rechte und linke Sägezähne in gleichmäßiger Abfolge angeordnet.

Aus der DE-A 44 27 204 ist ein Kreissägeblatt vorbekannt, welches für nicht-medizinische Zwecke verwendbar und in beiden Drehrichtungen einsetzbar ist. Dabei sind paarweise Hartmetall-Schneideinsätze angeordnet, die über die Ebene des Kreissägeblattes seitlich vorstehen.

Der Erfindung liegt die Aufgabe zugrunde, ein chirurgisches Sägeblatt der eingangs genannten Art zu schaffen, welches bei einfachem Aufbau und kostengünstiger Herstellbarkeit ein optimiertes Schnittverhalten zeigt und ohne wesentliche Schädigung von umgebendem Knochenmaterial eine präzise Schnittführung gestattet.

Erfindungsgemäß wird die Aufgabe durch die Merkmale des Hauptanspruches gelöst, die Unteransprüche zeigen weitere vorteilhafte Ausgestaltungen der Erfindung.

Das erfindungsgemäße Sägeblatt zeichnet sich durch eine Reihe erheblicher Vorteile aus.

Erfindungsgemäß ist vorgesehen, dass die Verzahnung jeweils Paare von zwei unmittelbar benachbarten Zähnen umfasst, welche, bezogen auf eine zur Mittelebene des Körpers senkrechte Ebene, symmetrisch zueinander ausgebildet sind. Durch diese symmetrische Ausgestaltung der Zähne erfolgt auch eine gleichmäßige Krafteintragung in die Verzahnung, so dass sich diese sehr gut zentriert und zu einem sehr ruhigen Schnittverlauf führt. Es ist somit möglich, das Sägeblatt gezielter zu führen und präzisere Knochenschnitte zu erzeugen.

Die genannten Vorteile sind sowohl bei einem Freihand-Einsatz des Sägeblattes als auch beim Einsatz in speziell hierfür vorgesehenen Schablonen vorhanden.

Da das Sägeblatt oszillierend hin und her bewegt wird, gelangen die einzelnen Zähne der Gruppen oder Paare jeweils gleichmäßig zum Einsatz, wodurch sich eine hervorragende, gleichmäßige Spanbildung ergibt.

Besonders günstig ist es, wenn jeder Zahn in Form eines in der Seitenansicht ein gleichschenkliges oder schiefwinkliges Dreieck bildenden Körpers ausgestaltet ist, an dessen einer Kante einseitig eine Fase angeschliffen ist. Somit kommen alternierend zu unterschiedlichen Seiten des Körpers des Sägeblattes die Schneidkanten zum Einsatz. Dies führt zu einem ruhigen Schnitt und zu einer Verhinderung von Rattermarken oder Ähnlichem.

Die Fasen jedes Paare von Zähnen können bevorzugterweise voneinander wegweisend ausgebildet sein. Hierdurch ist das Sägeblatt in beiden Schnittrichtungen in gleicher Weise einsetzbar.

Die erfindungsgemäß vorgesehenen Paare von Zähnen sind jeweils alternierend zu der Mittelebene des Körpers des Sägeblattes angeordnet. Dies bedeutet, dass einmal "linke" Paare von Zähnen zum Einsatz kommen, gefolgt von Paaren von "rechten" Zähnen. Auch hierdurch erhöht sich die Präzision der Schnittführung sowie die ruhige Handhabung des Sägeblattes.

Besonders günstig kann es sein, wenn zwischen zwei benachbarten Paaren von Zähnen zumindest ein weiterer Zahn angeordnet ist. Dieser kann über zusätzliche, veränderte Schnittgeometrien erfolgen, um zum einen die Zentrierung zu verbessern und um zum anderen Knochenbereiche abzutragen, die von den beiden aufeinander folgenden Paaren von Zähnen nicht in optimaler Weise bearbeitbar sind.

Der zumindest eine weitere Zahn kann dieselbe Höhe aufweisen wie die benachbarten Paare von Zähnen, es ist jedoch auch möglich, eine unterschiedliche Höhe vorzusehen. Es kann beispielsweise vorteilhaft sein, den weiteren Zahn höher oder niedriger auszubilden. Weiterhin ist es möglich, den weiteren Zahn mit einer Querschneide zu versehen, so dass sich für diesen Zahn eine andere Schnittgeometrie ergibt.

In einer Weiterbildung der Erfindung ist es auch möglich, zwischen den benachbarten Paaren von Zähnen zwei weitere Zähne anzuordnen, Hierbei kann es günstig sein, wenn der sich an ein Paar von Zähnen anschließende Zahn gleich dem jeweils folgenden Zahn des nächsten Paares ausgebildet ist. Auch hierbei werden beim Einsatz des Sägeblattes in einer hin und her schwingenden Bewegung zwischen den einzelnen Paaren zusätzliche Zähne zum Einsatz gebracht, die zu einer veränderten Schnittgeometrie beitragen. Bei der letztgenannten Ausgestaltungsvariante werden keine neuen Schnittgeometrien eingefügt, vielmehr werden lediglich Zahnformen wiederholt, die dem jeweils nachfolgenden Paar von Zähnen gleich sind. Somit vereinfacht sich auch die Herstellung des Sägeblattes ganz erheblich.

Im Folgenden wird die Erfindung anhand von Ausführungsbeispielen in Verbindung mit der Zeichnung beschrieben. Dabei zeigt:
- Fig. 1: eine schematische Seitenansicht eines Ausführungsbeispiels eines erfindungsgemäßen Sägeblatts,
- Fig. 2: eine vergrößerte Detailansicht eines ersten Paares erfindungsgemäßer Zähne,
- Fig. 3: eine Gesamtdarstellung eines Aufbaus der erfindungsgemäßen Verzahnung nach einem ersten Ausführungsbeispiel unter Verwendung des in Fig. 2 gezeigten Zahnpaares,
- Fig. 4: eine vergrößerte Detaildarstellung einer alternativen Ausgestaltung der Verzahnung,
- Fig. 5: eine Darstellung der Gesamtverzahnung unter Verwendung des in Fig. 4 gezeigten Details,
- Fig. 6: eine vergrößerte Teil-Darstellung eines weiteren Ausführungsbeispiels der erfindungsgemäßen Verzahnung,
- Fig. 7: eine Gesamtdarstellung des Ausführungsbeispiels der Verzahnung gemäß der Detailansicht von Fig. 6, und
- Fig. 8: eine exemplarische Darstellung einer durch seitliche Hohlkehle modifizierten Verzahnung.

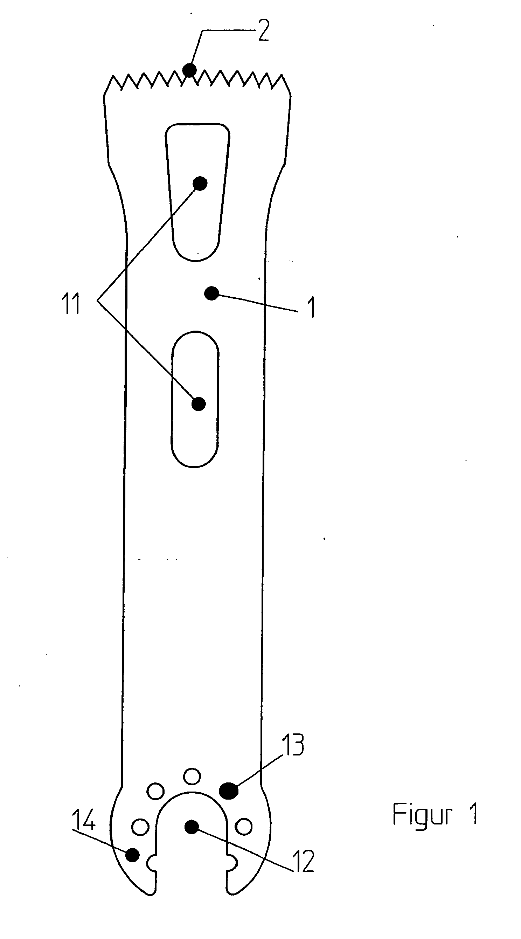

Durch die Fig. 1 ist in der Seitenansicht ein Sägeblatt dargestellt, welches einen im Wesentlichen flachen, plattenförmigen Körper 1 aufweist, der an seinem Randbereich mit einer bogenförmigen Verzahnung 2 versehen ist. An den Körper 1 schließt sich ein im Wesentlichen kreisrunder Einspannbereich 14 an, welcher mit Montageausnehmungen 12, 13 versehen ist. Die Dimensionierung und Ausgestaltung des Körpers 1 sowie der Montageausnehmungen 12 und 13 und des Einspannbereiches 14 entsprechen dem Stand der Technik und können den jeweiligen Einsatzbedingungen angepasst werden. Auf eine detaillierte Beschreibung kann an dieser Stelle verzichtet werden.

Die Verzahnung 2 ist bevorzugterweise bogenförmig ausgestaltet, damit bei einer schwingenden, hin und her gehenden oszillierenden Bewegung um die Mittelachse der Montageausnehmung 12 ein gleichmäßiger Schnitt erfolgen kann.

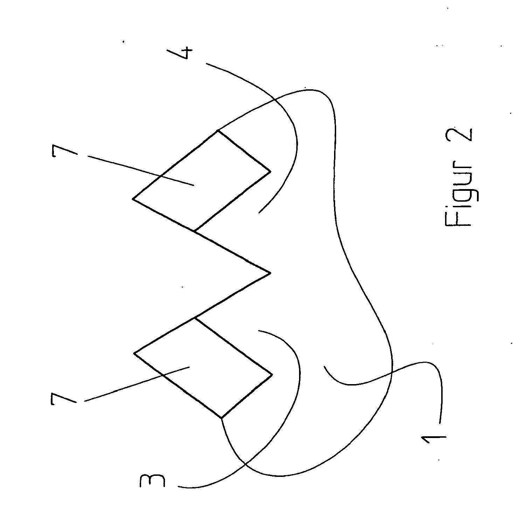

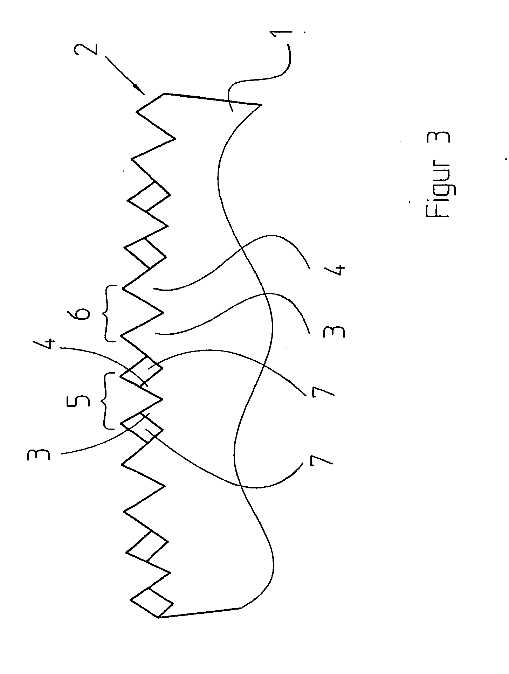

Die Fig. 2 und 3 zeigen ein Ausführungsbeispiel der erfindungsgemäßen Verzahnung. Diese umfasst Paare 5, 6 mit jeweils zwei Zähnen 3, 4. Die Fig. 2 zeigt in vergrößerter Detailansicht ein derartiges Paar von Zähnen 3, 4. Die Zähne sind zu einer senkrecht zur Mittelebene des Körpers 1 angeordneten Symmetrieachse symmetrisch ausgebildet, sie weisen jeweils an der abgewandten Seite eine Fase 7 auf, durch welche die entsprechenden Schneidkanten gebildet werden. Die beiden Zähne jedes Paares 5, 6 sind jeweils gleich hoch, sie sind im Wesentlichen aus einem nicht dargestellten Zahnrohling, der die Form eines gleichschenkligen oder schiefwinkligen Dreiecks bildet, erzeugt worden.

Wie sich aus Fig. 3 ergibt, sind alternierend jeweils Paare 5, 6 von Zähnen 3, 4 vorgesehen. Die Paare von Zähnen sind jeweils gleich, ihre Ausrichtung ist jedoch, bezogen auf die nicht dargestellte Mittelebene des Körpers 1, alternierend gewählt, so dass die Fasen des einen Paares 5 zu einer Seite des Körpers 1 weisen, während die Fasen des anderen Paares 6 zur anderen Seite des Körpers 1 gerichtet sind.

Bedingt durch den symmetrischen Aufbau der beiden Zähne 3, 4 eines Paares und bedingt durch die Abfolge von jeweils symmetrisch zueinander angeordneten Paaren ergibt sich ein sehr gleichmäßiger Schnitt mit gleichmäßig auf den Schnittbereich aufzubringenden Kräften. Hierdurch werden sowohl Schwingungen als auch Abwanderungen oder Dezentrierungen des Sägeblattes während des Schnittes verhindert.

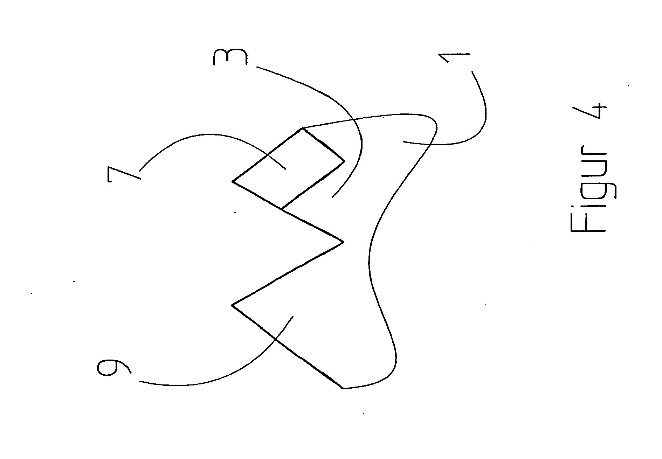

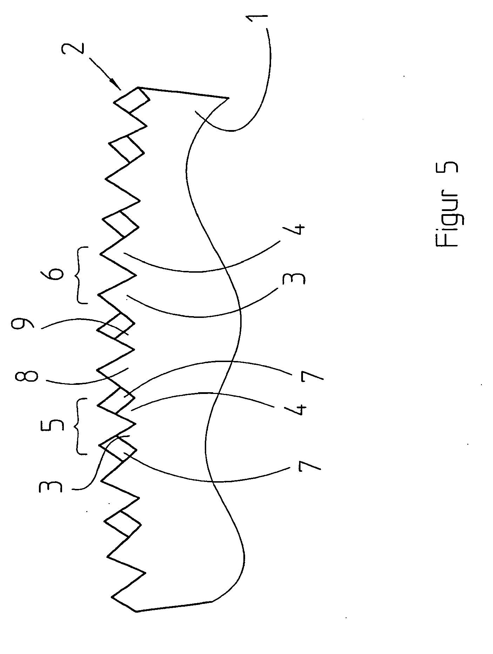

Die Fig. 4 und 5 zeigen ein abgewandeltes Ausführungsbeispiel, bei welchem zwischen den einzelnen Paaren 5, 6 jeweils weitere Zähne 8, 9 angeordnet sind. Die Fig. 4 zeigt eine vergrößerte Detaildarstellung hierzu.

Wie sich insbesondere aus der Fig. 5 ergibt, sind jeweils gleiche Zähne verwendet, auch für die weiteren Zähne 8, 9. Die Anordnung und Ausrichtung der weiteren Zähne 8, 9 erfolgt so, dass der sich jeweils an ein Paar 5 anschließende weitere Zahn 8 gleich ist zu dem ersten Zahn 3 des nachfolgenden Paares 6. In entsprechender Weise ist der sich an den Zahn 3 des Paares 6 anschließende weitere Zahn 9 gleich ausgebildet zu dem ersten Zahn 4 des nachfolgenden Paares 5 von Zähnen.

Die weiteren Zähne 8, 9 können dieselbe Höhe aufweisen wie die Zähne 3, 4 des jeweiligen Paares 5 bzw. 6. Es ist jedoch auch möglich, die weiteren Zähne 8, 9 mit einer größeren Höhe oder einer kleineren Höhe vorzusehen.

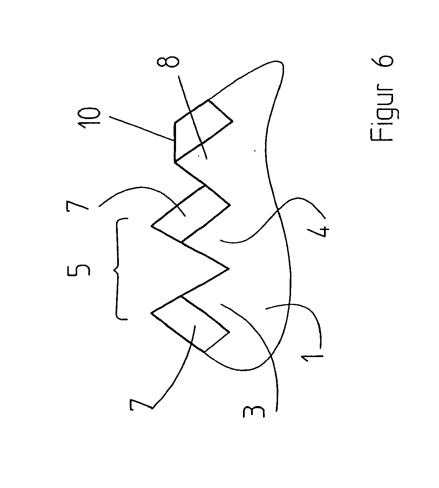

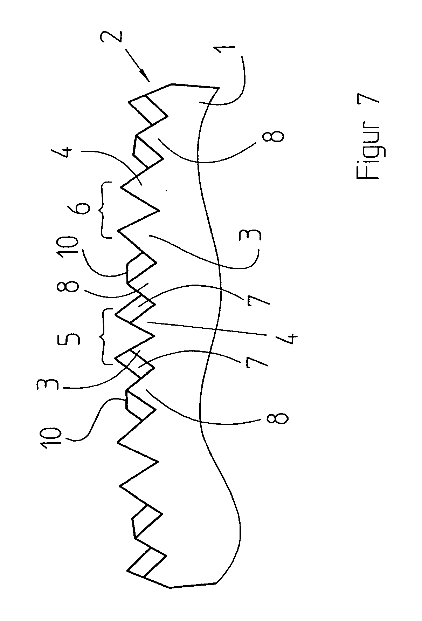

Aus den Fig. 6 und 7 ist ein weiteres Ausführungsbeispiel ersichtlich, bei welchem zwischen den benachbarten Paaren 5 und 6 jeweils nur ein weiterer Zahn 8 angeordnet ist. Dieser ist in unterschiedlicher Geometrie ausgestaltet. Bei dem gezeigten Ausführungsbeispiel ist er, wie sich auch aus der vergrößerten Darstellung der Fig. 6 ergibt, niedriger ausgebildet und mit einer Querschneide 10 versehen.

Die erfindungsgemäße Verzahnung führt, wie bereits erwähnt, zu einem präzisen Sägeschnitt, welcher sehr effizient ist. Die Schnittbreite entspricht mindestens der Sägeblattstärke.

Insbesondere bei dem in den Fig. 6 und 7 gezeigten Ausführungsbeispiel führt die Anordnung der Paare 5, 6 von Zähnen 3, 4 zu einer guten Zentrierung, während der etwas niedrigere weitere Zahn 8 durch seine Querschneide 10 zu einem besonders effizienten Materialabtrag beiträgt.

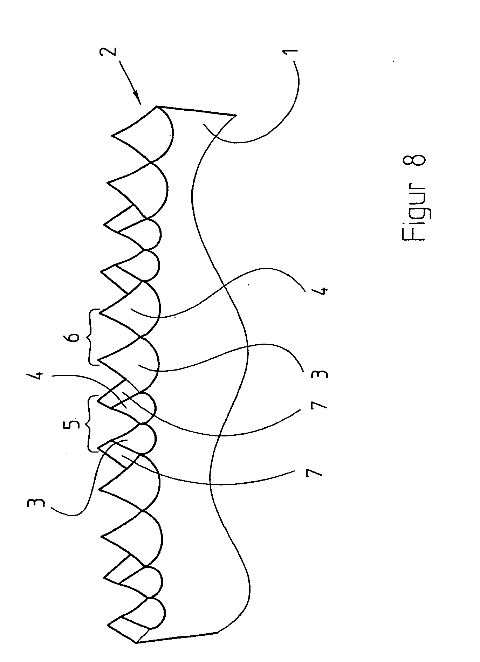

Die Fig. 8 zeigt eine Ausgestaltungsform, ähnlich der Darstellung der Fig. 7, bei welcher sämtliche Zähne mit seitlichen Hohlkehlen versehen sind. Es versteht sich, dass auch einzelne Gruppen mit derartigen seitlichen Hohlkehlen ausgebildet sein können. Weiterhin versteht es sich, dass die Hohlkehlen nicht beidseitig an den Zähnen vorgesehen sein müssen, vielmehr ist es möglich, die einzelnen Gruppen jeweils nur an einer Seite mit einer Hohlkehle zu versehen, die Hohlkehlen abwechselnd anzuordnen oder eine alternierende Ausgestaltung zu wählen.

Es versteht sich, dass auch einzelne Gruppen oder einzelne Zähne geschränkt werden können, während andere Gruppen oder andere Zähne ungeschränkt ausgebildet sind.

## Patentansprüche

1. Chirurgisches Sägeblatt für oszillierende Säge mit einem flachen, plattenförmigen Körper (1), welcher an zumindest einem Kantenbereich mit einer Verzahnung (2) versehen ist, welche eine Vielzahl von ungeschränkten Zähnen umfasst, wobei die Verzahnung (2) jeweils Paare (5, 6) von zwei unmittelbar benachbarten Zähnen (3, 4) umfasst, **dadurch gekennzeichnet, dass** die zwei unmittelbar benachbarten Zähne (3, 4), bezogen auf eine zur Mittelebene des Körpers (1) senkrechte Ebene, symmetrisch zueinander ausgebildet sind und dass die Paare (5, 6) von Zähnen (3, 4) jeweils alternierend zu der Mittelebene des Körpers (1) angeordnet sind.

2. Sägeblatt nach Anspruch 1, **dadurch gekennzeichnet, dass** jeder Zahn (3, 4) in Form eines in der Seitenansicht ein gleichschenkliges oder schiefwinkligen Dreieck bildenden Körpers ausgebildet ist, an dessen einer Kante einseitig eine Fase (7) angeschliffen ist.

3. Sägeblatt nach Anspruch 2, **dadurch gekennzeichnet, dass** die Fasen (7) jedes Paares (5, 6) von Zähnen (3, 4) voneinander wegweisend ausgebildet sind.

4. Sägeblatt nach einem der Ansprüche 1 bis 3, **dadurch gekennzeichnet, dass** zwischen zwei benachbarten Paaren (5, 6) von Zähnen (3, 4) zumindest ein weiterer Zahn (8, 9) angeordnet ist.

5. Sägeblatt nach Anspruch 4, **dadurch gekennzeichnet, dass** der zumindest eine weitere Zahn (8, 9) dieselbe Höhe wie die Zähne (3, 4) des Paares (5, 6) aufweist.

6. Sägeblatt nach Anspruch 4, **dadurch gekennzeichnet, dass** der zumindest eine weitere Zahn (8, 9) eine unterschiedliche Höhe wie die Zähne (3, 4) des Paares (5, 6) aufweist.

7. Sägeblatt nach Anspruch 6, **dadurch gekennzeichnet, dass** der zumindest eine weitere Zahn (8, 9) höher ausgebildet ist.

8. Sägeblatt nach Anspruch 6, **dadurch gekennzeichnet, dass** der zumindest eine weitere Zahn (8, 9) niedriger ausgebildet ist.

9. Sägeblatt nach einem der Ansprüche 4 bis 8, **dadurch gekennzeichnet, dass** der zumindest eine weitere Zahn (8) mit einer Querschneide versehen ist.

10. Sägeblatt nach einem der Ansprüche 4 bis 9, **dadurch gekennzeichnet, dass** zwischen den beiden benachbarten Paaren (5, 6) von Zähnen (3, 4) zwei weitere Zähne (8, 9) angeordnet sind, wobei der sich an ein Paar (5, 6) von Zähnen anschließende Zahn (8, 9) gleich dem jeweils folgenden Zahn (3, 4) des nächsten Paares (5, 6) ausgebildet ist.

11. Sägeblatt nach einem der Ansprüche 4 bis 10, **dadurch gekennzeichnet, dass** einzelne bzw. alle Schneiden seitlich durch Hohlkehlen modifiziert sind.

## Claims

1. Surgical saw blade for an oscillating saw, with a flat, plate-shaped body (1), provided on at least one edge area with a set of teeth (2) comprising a multiplicity of non-set teeth, wherein the set of teeth (2) comprises in each case pairs (5, 6) of two immediately adjacent teeth (3, 4), **characterised in that** the two immediately adjacent teeth (3, 4) are constructed symmetrically to one another), related to a plane perpendicular to the centre plane of the body (1), and **in that** the pairs (5, 6) of teeth (3, 4) are arranged alternating to the centre plane of the body (1) in each case.

2. Saw blade according to claim 1, **characterised in that** each tooth (3, 4) is constructed in the form of a body forming in side view an isosceles or oblique-angled triangle, on one edge of which a chamfer (7) is ground on one side.

3. Saw blade according to claim 2, **characterised in that** the chamfers (7) of each pair (5, 6) of teeth (3, 4) are constructed pointing away from one another.

4. Saw blade according to one of claims 1 to 3, **characterised in that** at least one further tooth (8, 9) is arranged between two adjacent pairs (5, 6) of teeth (3, 4).

5. Saw blade according to claim 4, **characterised in that** the at least one further tooth (8, 9) has the same height as teeth (3, 4) of pair (5, 6).

6. Saw blade according to claim 4, **characterised in that** the at least one further tooth (8, 9) has a different height from teeth (3, 4) of pair (5, 6).

7. Saw blade according to claim 6, **characterised in that** the at least one further tooth (8, 9) is constructed as higher.

8. Saw blade according to claim 6, **characterised in that** the at least one further tooth (8, 9) is constructed as lower.

9. Saw blade according to one of claims 4 to 8, **characterised in that** the at least one further tooth (8) is provided with a cross edge.

10. Saw blade according to one of claims 4 to 9, **characterised in that** two further teeth (8, 9) are arranged between the two adjacent pairs (5, 6) of teeth (3, 4), wherein the tooth (8, 9) adjoining one pair (5, 6) of teeth is constructed as identical to the tooth (3, 4) of the next pair (5, 6) following in each case.

11. Saw blade according to one of claims 4 to 10, **characterised in that** individual or all the edges are modified laterally by grooves.

## Revendications

1. Lame de scie chirurgicale pour scie oscillante, comportant un corps (1) plat en forme de plaque, qui est muni au moins sur un bord d'une denture (2) présentant une pluralité de dents non avoyées, la denture (2) comportant respectivement des paires (5, 6) de deux dents (3, 4) directement adjacentes, **caractérisée en ce que** les deux dents (3, 4) directement adjacentes, par rapport à un plan perpendiculaire au plan médian du corps (1), sont réalisées symétriquement l'une à l'autre, et **en ce que** les paires (5, 6) de dents (3, 4) sont disposées respectivement en alternance par rapport au plan médian du corps (1).

2. Lame de scie selon la revendication 1, **caractérisée en ce que** chaque dent (3, 4) est réalisée sous la forme d'un corps, qui forme sur la vue de profil un triangle isocèle ou scalène et dont une arête est réalisée sur un côté avec un chanfrein (7).

3. Lame de scie selon la revendication 2, **caractérisée en ce que** les chanfreins (7) de chaque paire (5, 6) de dents (3, 4) sont réalisés en s'écartant l'un de l'autre.

4. Lame de scie selon l'une quelconque des revendications 1 à 3, **caractérisée en ce qu'**au moins une dent (8, 9) supplémentaire est située entre deux paires (5, 6) adjacentes de dents (3, 4).

5. Lame de scie selon la revendication 4, **caractérisée en ce que** ladite au moins une dent (8, 9) supplémentaire présente la même hauteur que les dents (3, 4) de la paire (5, 6).

6. Lame de scie selon la revendication 4, **caractérisée en ce que** ladite au moins une dent (8, 9) supplémentaire présente une hauteur différente de celle des dents (3, 4) de la paire (5, 6).

7. Lame de scie selon la revendication 6, **caractérisée en ce que** ladite au moins une dent (8, 9) supplémentaire est plus haute.

8. Lame de scie selon la revendication 6, **caractérisée en ce que** ladite au moins une dent (8, 9) supplémentaire est plus basse.

9. Lame de scie selon l'une quelconque des revendications 4 à 8, **caractérisée en ce que** ladite au moins une dent (8) supplémentaire est munie d'un tranchant transversal.

10. Lame de scie selon l'une quelconque des revendications 4 à 9, **caractérisée en ce que** deux dents supplémentaires (8, 9) sont situées entre les deux paires (5, 6) adjacentes de dents (3, 4), la dent (8, 9) dans le prolongement d'une paire (5, 6) de dents est réalisée à l'identique de la dent (3, 4) directement suivante de la paire (5, 6) suivante.

11. Lame de scie selon l'une quelconque des revendications 4 à 10, **caractérisée en ce que** certains tranchants ou tous les tranchants sont modifiés sur le côté par des gorges creuses.
